# EUROPEAN PATENT APPLICATION

(11) **EP 4 670 716 A1**
(43) Date of publication of application: **31.12.2025**
(21) Application number: 24760125.5
(22) Date of filing: 07.02.2024
(51) Int. Cl.: A61K 31/365, A61P 35/00, A61P 37/04, A61P 43/00

(54) **DENDRITIC CELL ACTIVATOR**

(30) Priority: 24.02.2023 JP 2023027142
(71) Applicant: Kyushu University, National University Corporation, Nishi-ku Fukuoka-shi Fukuoka 819-0395 (JP); MITSUI NORIN CO., LTD., Tokyo 105-8427 (JP)
(72) Inventor: TACHIBANA Hirofumi, Fukuoka-shi, Fukuoka 819-0395 (JP); TANAKA Yuko, Fujieda-shi, Shizuoka 426-0133 (JP); TOMIOKA Reno, Fujieda-shi, Shizuoka 426-0133 (JP)
(74) Representative: Gleiss Große Schrell und Partner mbB
(86) International application number: PCT/JP2024/004033
(87) International publication number: WO 2024/176823

(57) **Abstract**

Provided is a dendritic cell activator that contains, as an active ingredient, a component derived from a natural product which has been eaten for many years and the safety of which has been empirically confirmed, and that is used for inducing or promoting an acquired immune response effective against cancer or infectious disease. Specifically provided are: a dendritic cell activator containing, as an active ingredient, 5-(3,5-dihydroxyphenyl)-y-valerolactone represented by formula (I); and a medicine, a supplement, and a food or beverage each containing the compound represented by formula (I) as an active ingredient.

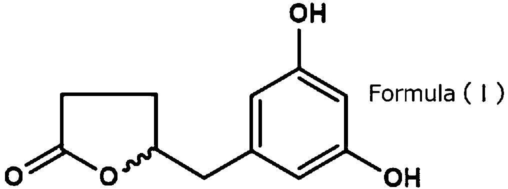

[The configuration of the wavy line may be either R-form or S-form.]

## Description

### Technical Field

The present invention relates to a dendritic cell activator containing, as an active ingredient, 5-(3,5-dihydroxyphenyl)-γ-valerolactone represented by formula (I) (hereinafter also described as EGC-M5). The purpose of the present invention is to improve the immune function to prevent or treat the diseases including cancer and viral diseases. The present invention relates to a medicine, a supplement, and a food or beverage each using the dendritic cell activator.

### Background Art

The immune regulatory functions of the innate immunity and the acquired immunity are exerted in a complex manner within the body and play an important role in maintaining daily health. For this reason, the activation of the immune function is recognized as being particularly important for treating and preventing diseases and promoting health. Regarding the substances that promote the immune activation in the body, various immune-activating substances have been found so far. However, many side effects are already known, some of which occur frequently and some of which are severe, posing challenges when performing treatment. Thus, when healthy individuals ingest such substances for the purpose of disease prevention, immune-activating substances derived from natural products that have a low risk of side effects and can be ingested on a daily basis are preferable, and research into immune-activation using food ingredients is ongoing.

A wide range of health functions have been reported for catechin, a type of plant polyphenol. The functional properties of several compounds that are intestinal bacterial metabolites of catechins have been evaluated and reported.

The immune system, which is a defense mechanism of the body, plays a role in recognizing, as antigens, and eliminating pathogens such as bacteria and viruses that invade from the outside, as well as tumor cells that develop within the body. The innate immune system is an immune system that is present from birth, whereas the acquired immune system is formed in response to stimulation by foreign antigens after birth.

When an antigen (pathogen or tumor) invades or develops in the body, macrophages, natural killer cells, and dendritic cells, which work as innate immune system, first work to eliminate the antigen. Next, the dendritic cells induce differentiation into the killer T cells (CD8⁺ T cells) that specifically attack the antigen and antibody production by presenting the captured antigen information to T cells and B cells, thereby activating the acquired immune system.

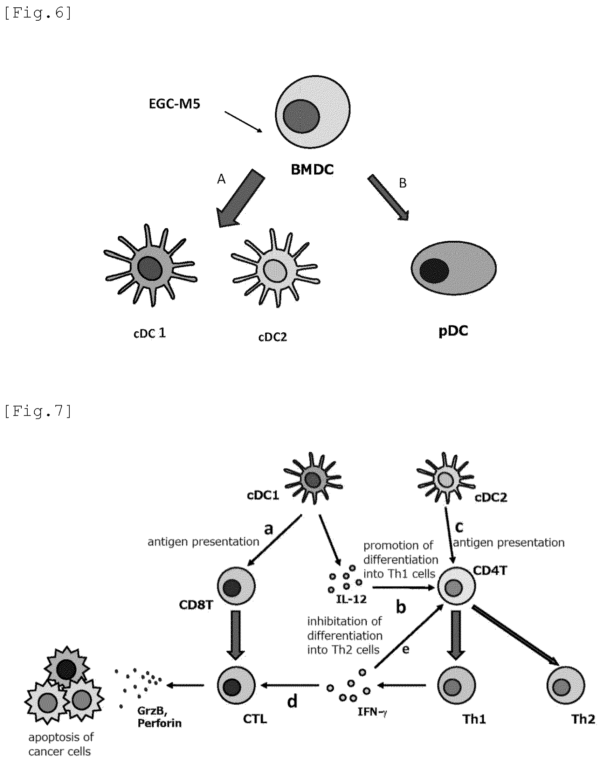

The dendritic cells (DCs) play an important role in bridging the innate immune system and the acquired immune system. DC precursor cells (Bone marrow-derived dendritic cells: BMDCs) derived from hematopoietic stem cells in the bone marrow differentiate into plasmacytoid DCs (plasmacytoid dendritic cells: pDCs) (arrow B in Fig.6) or into conventional DCs (conventional dendritic cells: cDCs) (arrow A in Fig.6) to function as DCs.

Although the plasmacytoid DCs (pDCs) have a weak antigen-presenting ability, they have the effect of secreting large amounts of type I interferon-α and -β (IFN-α, β) in response to viral infection. In addition, pDCs play a role in inhibiting excessive immune responses by inducing the differentiation of naive CD4 positive T cells (hereinafter described as naive CD4⁺ T cells) into IL-10-producing T cells and regulatory T cells (Tregs), thereby inhibiting the onset of autoimmune diseases, inflammatory diseases, allergic reactions, and the like. For this reason, it has been reported that lactic acid bacteria belonging to Lactobacillus helveticus or cultures thereof (Patent Document 1) and a milk fermentation product of Lactobacillus kefiri, or processed product thereof which enhances the ability of DCs to produce immunoregulatory cytokines (Patent Document 2) are highly safe and useful as preventive or therapeutic agents for viral infections by inducing proliferation of pDCs. However, while strengthening acquired immune function via pDCs can inhibit excessive immune responses (inflammation) and prevent secondary tissue damage by inducing regulatory T cells (Tregs) and promoting the production of the immunoregulatory cytokine IL-10 by pDCs, it can also cause a decrease in cytotoxic activity against antigens. In addition, it has been reported that pDCs accumulate in the bone marrow of multiple myeloma patients and induce immunosuppression and tumor promotion (Non-Patent Document 1), and that since proliferating and activating Treg cells enhance the immunosuppression, there is an association with poor prognosis in some cancer types (Non-Patent Document 2). Thus, strengthening the acquired immune system targeting only pDCs may have the disadvantage of reducing tumor elimination efficiency and contributing to the promotion of tumor growth.

On the other hand, after being activated, the conventional DCs (cDCs) are distributed in lymphatic tissues (spleen, lymph nodes, bone marrow, and the like) and non-lymphatic tissues (lungs, skin), and cDC1 has the effect of inducing CD8⁺ T cells (described as CD8T in Fig.7) into cytotoxic T cells (CTLs) by the cross-presentation pathway through MHC class I receptors (arrow a in Fig.7), promoting the differentiation into Th1 cells by secreting IL-12 (arrow b in Fig.7), and activating natural killer cells (NK cells) and natural killer T cells (NKT cells). cDC2 has the effect of activating CD4⁺ T cells (helper T cells; described as CD4T in Fig.7) by presenting antigens through MHC class II receptors (arrow c in Fig.7), and promotes the differentiation into Th1 cells, Th2 cells, or the like depending on the cytokine environment of the place where antigens are presented. When CD4+ T cells are presented with an antigen in the presence of IL-12, the differentiation into Th1 cells is promoted. Furthermore, Th1 cells secrete IFN-γ, which enhances cytotoxic activity of the cytotoxic T cells (CTLs) (arrow d in Fig.7). Hence, strengthening the acquired immune system by promoting the differentiation of hematopoietic stem cells to the conventional DCs (cDCs) accompanied by activation of cDCs (arrow A in Fig.6) contributes to the effective antigen elimination against not only tumor cells but also antigens such as host cell parasitic microorganisms (bacteria, fungi, protozoa) and viruses that invade from the outside, i.e., the acquisition of antigen-specific cytotoxic activity, and is expected to reduce disease risk and enhance immune response functions.

### Citation List

### Patent Documents

### Patent Document

Patent Document 1: JP 2017-031109 A
Patent Document 2: JP 2017-007983 A

Non-Patent Document 1: Cancer Cell, 2009, Vol.16, p.309-323
Non-Patent Document 2: Cancer Res., 2012, Vol.72, p.5240-5249

### Summary of Invention

### Technical Problem

The problem of the present invention is to provide a dendritic cell activator that contains, as an active ingredient, a component derived from a natural product which has been eaten for many years and the safety of which has been empirically confirmed, and that is used for inducing or promoting an immune response effective against cancer or infectious disease or a medicine, a supplement, and a food or beverage for activating the dendritic cell.

### Solution to Problem

The present inventors focused on the strengthening of the acquired immune system through the differentiation into cDCs, which is accompanied by the activation of cDCs, and conducted an intensive search for the effect of the differentiation into cDCs and the activation of cDCs with regard to the components derived from the natural products, which have a long history of consumption and whose safety has been empirically confirmed, and found that an intestinal bacterial degradation product of catechins (formula (I)) exhibits an excellent effect of activating both cDC1s and cDC2s. The present inventors also found that since the cDCs activation by this component does not involve a reaction that inhibits cellular immunity (Th2 cell induction) when presenting antigens to T cells, this component effectively exhibits the effect of enhancing the ability of the acquired immune system to eliminate antigens (cytotoxic activity) leading to the completion of this invention.

That is, the present invention relates to:
[1] A dendritic cell activator characterized by containing, as an active ingredient, 5-(3,5-dihydroxyphenyl)-γ-valerolactone represented by formula (I): 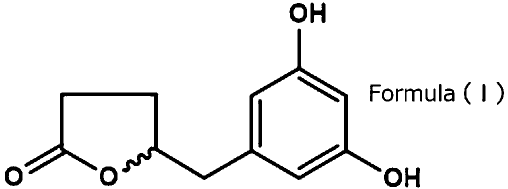 wherein in formula (I) the configuration of the wavy line may be either R-form or S-form.
[2] The dendritic cell activator according to item [1] used for promoting differentiation into conventional dendritic cells (cDCs).
[3] The dendritic cell activator according to item [2], wherein promotion of the differentiation into conventional DCs (cDCs) is accompanied by activation of cDCs.
[4] The dendritic cell activator according to item [2], wherein the dendritic cell activator promotes differentiation of CD4⁺ T cells into Th1 cells and inhibits differentiation of CD4⁺ T cells into Th2 cells.
[5] The dendritic cell activator according to item [2], wherein the dendritic cell activator promotes differentiation of CD8⁺ T cells into cytotoxic T cells (CTLs) and enhances cytotoxic activity.
[6] The dendritic cell activator according to any one of items [1] to [5], wherein the dendritic cell activator is an enhancer of acquired immune system.
[7] The dendritic cell activator according to item [6], wherein the acquired immune system is cellular immunity.
[8] The dendritic cell activator according to item [6], wherein the dendritic cell activator is an anticancer drug.

### Effects of the Invention

A dendritic cell activator containing 5-(3,5-dihydroxyphenyl)-γ-valerolactone as an active ingredient can enhance the acquired immune system by promoting the differentiation of hematopoietic stem cells into the conventional DCs (cDCs) accompanied by activation of cDCs. The dendritic cell activator containing 5-(3,5-dihydroxyphenyl)-γ-valerolactone as an active ingredient also contributes to the effective antigen elimination by cellular immunity against not only tumor cells but also antigens such as host cell parasitic microorganisms (bacteria, fungi, protozoa) and viruses that invade from the outside, i.e., the acquisition of antigen-specific cytotoxic activity, and is expected to reduce disease risk and enhance immune response functions.

### Brief Description of Drawings

[Fig.1] Fig.1 shows graphs that represent the proportion of each dendritic cell subset in bone marrow cells of mice intraperitoneally administered with EGC-M5.
[Fig.2] Fig.2 shows graphs that represent the proportion of each dendritic cell subset (Fig.2A), graphs that represent marker gene expression level of each dendritic cell (Fig.2B), and graphs that represent IL-12 gene expression level and production amount of IL-12 (Fig.2C) when EGC-M5 was added during induction of the differentiation of mouse bone marrow cells into the dendritic cells.
[Fig.3] Fig.3 shows graphs that represent the expression level of Th1 cell activation-related genes (Fig.3A), and a graph that represents the production amount of IFN-γ which is a cytokine secreted by Th1 cells (Fig.3B) when EGC-M5 was added to mouse splenic cells.
[Fig.4] Fig.4 shows graphs that represent the expression level of Th1 cell activation-related genes (Fig.4A) and Th2 cell activation-related genes (Fig.4B) in splenic cells of colon cancer model mice administered with EGC-M5 intraperitoneally.
[Fig.5] Fig.5A shows graphs that represent the expression level of CTL activation-related genes in the splenic cells of the colon cancer model mice administered with EGC-M5 intraperitoneally. Fig.5B shows a graph that represents the number of CD8⁺ T cells (CTLs) per unit of tumor tissue area.
[Fig.6] Fig.6 shows a schematic diagram of the predicted pathway of promoting action of differentiation into bone marrow-derived precursor dendritic cells (BMDCs) by EGC-M5.
[Fig.7] Fig.7 shows a schematic diagram of the predicted pathway of antitumor action by cDCs.

### Form to carry out invention

The dendritic cell activator of the present invention contains 5-(3,5-dihydroxyphenyl)-γ-valerolactone represented by the following formula (I) as an active ingredient. The dendritic cell activator can promote the differentiation of hematopoietic stem cells into the conventional DCs (cDCs) accompanied by activation of cDCs by containing, as an active ingredient, 5-(3,5-dihydroxyphenyl)-γ-valerolactone represented by following formula (I): 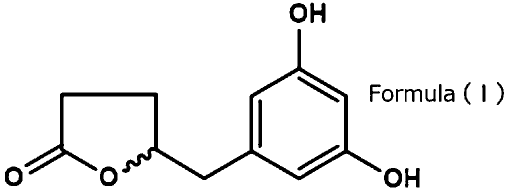 wherein in formula (I) the configuration of the wavy line may be either R-form or S-form.

Strengthening of the acquired immune system through promotion of the differentiation into cDCs by the dendritic cell activator of the present invention is described with reference to Fig.6 and 7.

Fig.6 shows a schematic diagram of the predicted pathway of promoting action of the differentiation into cDCs on bone marrow-derived precursor dendritic cells (BMDCs) by EGC-M5. Bone marrow-derived precursor dendritic cells (BMDCs) differentiate into the conventional DCs (cDCs) (arrow A in Fig.6) or the plasmacytoid DCs (pDCs) (arrow B in Fig.6). EGC-M5 which is an active ingredient of the dendritic cell activator of the present invention acts on bone marrow-derived precursor dendritic cells (BMDCs) and increases the proportion of conventional DCs (cDCs) among differentiated DCs, i.e., promotes the differentiation into cDCs.

Fig.7 shows a schematic diagram of the predicted pathway of antitumor action by cDCs. Immediately after differentiation and formation, cDCs have no encounter with foreign substances and express low levels of MHC class II molecules which are antigen-presenting molecules and auxiliary signal molecules. However, upon taking up antigen, cDCs change into mature cDCs that present antigens to naive T cells (CD8T and CD4T in Fig. 7), i.e., cDCs become activated. EGC-M5 which is an active ingredient of the dendritic cell activator of the present invention enhances antigen uptake, processing, and antigen presentation via MHC class I and II of cDCs, i.e., EGC-M5 activates cDCs. The activated cDCs present antigens to naive T cells (T cells that have never encountered an antigen; in Fig. 7 these correspond to CD8T and CD4T) via MHC class I and II, thereby activating naive T cells. cDCs are classified into two types: cDC1 which presents antigens via MHC class I and cDC2 which presents antigens via MHC class II. cDC1s activate naive CD8⁺ T cells (CD8T in Fig.7) and promote the differentiation of CD8T into cytotoxic T cells (CTLs) (arrow a in Fig.7), and promote the differentiation of naive CD4⁺ T cells (CD4T in Fig.7) into Th1 cells by secreting IL-12 (arrow b in Fig.7). cDC2s activate naive CD4⁺ T cells (CD4T in Fig.7) (arrow c in Fig.7) and promote the differentiation into Th1 cells in the presence of IL-12. Naive CD4⁺ T cells (CD4T in Fig.7) differentiate into Th1 cells due to IL-12 secretion by cDC1 and antigen presentation via MHC class II by cDC2 and Th1 cells secrete IFN-γ. IFN-γ further promotes the differentiation of naive CD4⁺ T cells into Th1 cells and inhibits the differentiation into Th2 cells which have the function of inhibiting the cellular immunity (arrow e in Fig.7). IFN-γ secreted by Th1 cells also enhances the cytotoxic activity of cytotoxic T cells (CTLs) (arrow d in Fig.7). Thus, the dendritic cell activator of the present invention strengthens the acquired immune system through promotion of the differentiation into cDCs.

In the present description, cell differentiation means that cells change into different cells by contact with other cells or by stimulation with a differentiation-inducing factor or the like.

Hematopoietic stem cells formed in the bone marrow differentiate into either myeloid stem cells or lymphoid stem cells. Myeloid stem cells differentiate into any one of red blood cells, white blood cells, platelets, granulocytes (neutrophils, eosinophils, basophils), monocytes/macrophages, or dendritic cells, while lymphoid stem cells differentiate into any one of lymphocytes (T cells, B cells, NK cells) which are a type of white blood cells.

In the present description, promotion of the differentiation induction of hematopoietic stem cells into the conventional dendritic cells (cDCs) (arrow A in Fig.6) means increasing the proportion of the conventional dendritic cells (cDCs) among dendritic cells differentiated from hematopoietic stem cells via myeloid stem cells. "Promotion of differentiation" in the present invention has the same meaning as "differentiation promotion" and "differentiation induction."

In the present description, activation of the conventional DCs (cDCs) means that immature cDCs that have just been differentiated (which have never encountered a foreign substance and have low expression levels of auxiliary signal molecules such as MHC class II molecules) change into mature cDCs. By changing into mature cDCs, cDCs come to express a large amount of MHC class II that presents antigenic peptides derived from viruses and tumors, at the same time increase the expression of auxiliary signal molecules, migrate to the T cell area of lymph nodes, and express the function of presenting antigens to naive T cells.

In the present description, cellular immunity has the same meaning as T cell-mediated immunity, which functions through the direct action of T cells, and is different from immunity induced by the action of antibodies (humoral immunity). Cellular immunity means an immune response characterized by directly attacking abnormal cells such as microorganisms (bacteria, fungi, protozoa) that are parasitic within host cells, virus-infected cells, and cancer cells.

The dendritic cell activator is not particularly limited as long as it is a dendritic cell activator containing 5-(3,5-dihydroxyphenyl)-γ-valerolactone as an active ingredient (hereinafter, also simply referred to as "dendritic cell activator"), and such dendritic cell activator may be in any form, for example, a liquid form such as an aqueous solution, a suspension, or an emulsion, a semi-solid form such as a gel or a paste, or a solid form such as a powder, granules, capsules, or tablets.

The dendritic cell activator may contain additives that are commonly used for formulation and are pharmaceutically acceptable, such as the excipients, the binders, the lubricants, the disintegrants, the preservatives, the isotonicity agents, the stabilizers, the dispersants, the antioxidants, the colorants, the flavorings, and the buffers.

The amount of the dendritic cell activator to be taken will vary depending on age, body weight, symptoms, therapeutic effect, administration method, treatment time, and the like, but typically, an adult can take the dendritic cell activator containing the compound which is an active ingredient in the range of 0.1 mg to 1000 mg, preferably 1 mg to 500 mg in a single dose, from once to several times a day orally or parenterally.

The dendritic cell activator can also be a medicine. The medicine is not particularly limited as long as it contains the compound of the formula (I) as an active ingredient and is a medicine for promoting the differentiation into the conventional DCs (cDCs) accompanied by activation of conventional CDs (hereinafter, also simply referred to as "medicine"). Examples of such medicine include medicines included in the Japanese Pharmacopoeia, and for treating or preventing cancers such as stomach cancer, colon cancer, lung cancer, prostate cancer, breast cancer, uterine cancer, esophageal cancer, liver cancer, and malignant lymphoma, and diseases caused by impaired immune function including allergies such as atopic dermatitis and hay fever, autoimmune diseases such as rheumatoid arthritis and psoriatic arthritis, blood diseases such as leukemia, and viral hepatitis such as hepatitis B and hepatitis C. The dendritic cell activator is preferably the medicines for treating or preventing cancer, i.e., anticancer agents. In addition, since the cellular immunity effectively works against antigens such as host cell parasitic microorganisms (bacteria, fungi, protozoa) and viruses that invade from the outside, the dendritic cell activator is also preferably the medicines for treating and preventing the diseases caused by these. For example, the dendritic cell activator is also preferably the medicines for treating or preventing diseases caused by all viruses such as influenza and herpes, intracellular parasitic bacteria (Mycobacterium tuberculosis, Salmonella typhi, Treponema pallidum, Salmonella, Mycoplasma, Chlamydia, Legionella, etc.), intracellular parasitic fungi (Candida, Cryptococcus, Aspergillus, etc.), and parasitic protozoa (Toxoplasma, Strongyloides stercoralis, etc.).

Examples of formulation include tablets, granules, fine granules, pills, powders, capsules, troches, chewable tablets, liquids (drinks), infusions, etc. For the topical medication, any form that can be absorbed into the body through the skin surface or mucous membranes may be used, examples of formulation include lotions, ointments, creams, gels, tapes, patches, aerosols, and inhalants.

The medicines may contain additives that are commonly used for formulation and are pharmaceutically acceptable, such as the excipients, the binders, the lubricants, the disintegrants, the preservatives, the isotonicity agents, the stabilizers, the dispersants, the antioxidants, the colorants, the flavorings, and the buffers.

The amount of the medicines to be administered will vary depending on age, body weight, symptoms, therapeutic effect, administration method, treatment time, etc., but typically, an adult can be administered with the medicines containing the compound of the formula (1) as an active ingredient in the range of 0.1 mg to 1000 mg, preferably 1 mg to 500 mg in a single dose, from once to several times a day orally or parenterally.

The dendritic cell activator may be used as quasi-drugs. The quasi-drug is not particularly limited as long as it is a quasi-drug designated by the Minister of Health, Labor and Welfare, and is used to prevent nausea and other discomfort, bad breath or body odor, to prevent heat rash, sores, etc., to prevent hair loss, to grow hair, or to remove hair. Examples of formulations of quasi-drugs include oral liquids, health drinks, vitamin-containing health supplements, tablets, granules, liquids, lotions, ointments, creams, gels, tapes, patches, and aerosols.

The supplement is not particularly limited as long as it is a supplement containing the compound of the formula (I) as an active ingredient and for promoting the differentiation into conventional DCs (cDCs) accompanied by activation of conventional DCs (hereinafter, also simply referred to as "supplement") and such supplement may be in any form, for example, a liquid form such as an aqueous solution, a suspension, or an emulsion, a semi-solid form such as a gel or a paste, or a solid form such as a powder, granules, capsules, or tablets.

The supplement may contain amino acids such as leucine and valine, minerals such as zinc and calcium, vitamins such as vitamin A, vitamin B1, B2, B6, B12, vitamin C, vitamin D, vitamin E, beta-carotene, and coenzyme Q10 and additives that are commonly used for formulation of the supplement, such as the excipients, the binders, the lubricants, the disintegrants, the preservatives, the isotonicity agents, the stabilizers, the dispersants, the antioxidants, the colorants, the flavorings, and the buffers.

The amount of the supplement to be taken will vary depending on age, body weight, symptoms, therapeutic effect, administration method, treatment time, etc., but typically, an adult can take the supplement containing the compound of the formula (1) as an active ingredient in the range of 0.1 mg to 1000 mg, preferably 1 mg to 500 mg in a single dose, from once to several times a day orally or parenterally.

The food and beverage of the present invention are not particularly limited as long as these are food and beverage containing the compound of the formula (I) as an active ingredient and for promoting the differentiation into conventional DCs (cDCs) accompanied by activation of conventional CDs (hereinafter, also simply referred to as "food and beverage in this case") and such food and beverage may be in any form, for example, a liquid form such as an aqueous solution, a suspension, or an emulsion, a semi-solid form such as a gel or a paste, or a solid form such as a powder, granules, capsules, or tablets.

Examples of food and beverage include instant foods (instant noodles, cup noodles, retort/prepared foods, preparatory canned foods, microwave foods, instant miso soup/clear soup, canned soup, freeze-dried foods, etc.); luxury drinks and luxury items such as carbonated drinks, citrus juice (grapefruit, orange, lemon, etc.), fruit juice drinks, soft drinks containing fruit juice, citrus pulp drinks, fruit drinks containing fruit pieces, vegetable drinks containing vegetables such as tomatoes, bell peppers, celery, cucumbers, carrots, potatoes, and asparagus, soy milk/soy milk drinks, coffee drinks, tea drinks, powdered drinks, concentrated drinks, sports drinks, nutritional drinks, alcoholic beverages, and tobacco; flour products such as macaroni/spaghetti, noodles, cake mixes, fried chicken flour, bread crumbs, and gyoza pastry; confectioneries such as caramel candies, chewing gum, chocolate, cookies/biscuits, cakes/pies, snacks/crackers, Japanese sweets/rice sweets/bean sweets, and dessert sweets; basic condiments such as soy sauce, miso (soybean paste), worcestershire sauces, processed tomato seasonings, mirin (sweet sake), vinegar, and sweeteners; composite seasonings and foods such as flavor seasonings, cooking mixes, curry bases, sauces, dressings, noodle soups and spices; fats and oils such as butter, margarine, mayonnaise, and vegetable oils; dairy products such as milk/ processed milk, dairy drinks, yogurt, lactic acid bacteria drinks, cheese, ice cream, formula milk, and cream; frozen foods such as ingredients frozen foods, semi-cooked frozen foods, and cooked frozen foods; canned seafood, canned fruit and pastes; processed seafood products such as fish ham and sausages, seafood paste products, seafood delicacies, dried seafood, and tsukudani (food boiled in soy sauce); canned livestock and pastes, canned meat; agricultural processed products such as canned fruit, jams and marmalade, pickles and boiled beans, dried agricultural goods, and cereals (processed grain products); baby food, and commercially available foods such as furikake and ochazuke nori (rice seasoning).

Taking advantage of the characteristics mentioned above of the dendritic cell activator, (a) the method for activating the dendritic cell activator characterized by administering the subject 5-(3,5-dihydroxyphenyl)-γ-valerolactone, (b) the immunostimulation method characterized by administering the subject 5-(3,5-dihydroxyphenyl)-γ-valerolactone, (c) the use of 5-(3,5-dihydroxyphenyl)-γ-valerolactone as the dendritic cell activator, the medicine, the supplement or the food and beverage and (d) the use of 5-(3,5-dihydroxyphenyl)-γ-valerolactone in the preparation of the dendritic cell activator, the medicine, the supplement or the food and beverage can be included.

Because 5-(3,5-dihydroxyphenyl)-γ-valerolactone which is the active ingredient of the dendritic cell activator, the medicine, the supplement or the food and beverage is the catechin metabolites, i.e., the compound generated from the catechins by the action and the like of the microorganisms which live in the living body of the mammals such as human, rat, mouse, and pig, the dendritic cell activator, the medicine, the supplement, and the food and beverage excellent in safety can be obtained by using the compound.

When the dendritic cell activator, the medicine, the supplement or the food and beverage contain the compound of the formula (I), these may contain the purified compound of the formula (I), or the composition containing the roughly purified compound of the formula (I).

The compound of the formula (I) can be obtained by the known organic chemical synthesis described in the following document (SYNTHESIS, 9, 1512-1520, 2010) and the like. For example, the compound of the formula (I) can be prepared using 3,5-(tert-butyldimethylsiloxy)bromobenzene as the substrate and using seven reactions including Swern oxidation and Wittig reaction.

The compound of the formula (I) also can be produced by the microbial transformation method using the intestinal microorganisms. When the compound of the formula (I) which is the catechin metabolites is produced by the microbial transformation method, the method include the method in which after the feces and the cecal content containing the intestinal microorganisms of rat or human is incubated to grow the intestinal microorganisms, the cultured microbial cells are suspended in buffer, physiological saline, water and the like, catechins to be used as the substrate are added in the suspension and the suspension is incubated.

Catechins to be added as the substrate include (+)-epigallocatechin, (-)-epigallocatechin, (-)-gallocatechin, which are non-gallate catechins, (-)-gallocatechingallate, and (-)-epigallocatechingallate, which are gallate catechins. (-)-epigallocatechin is preferable.

To obtain the compound of the formula (I), first of all catechins to be the substrate need to be transformed into the compound represented by following formula (II) by the microbial transformation. The preferable examples of the microorganisms having the ability to transform catechins into the compound represented by following formula (II) include Eggerthella lenta strain JCM9979, Adlercreutzia equolifaciens strain MT4s-5 (accession number FERM P-21738), and strain JCM14793, Asaccharobacter celatus strain JCM14811, and Slackia equolifaciens strain JCM16059 (See Biol. Pharm. Bull., 38, 325-330, 2015). 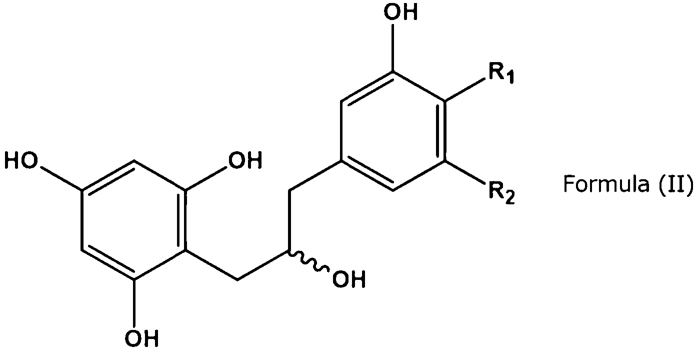 wherein in the formula (II), R₁ and R₂ independently represent hydroxy group (OH) or hydrogen atom (H) and the configuration of the wavy line may be either R-form or S-form.

Note that to obtain the compound represented by the formula (I), the compounds represented by the formula (II) in which R₁ is H and R₂ is OH need to be obtained. In that case, hydrogen and/or formic acid need to be added in the culture medium. When hydrogen and/or formic acid are not added in the culture medium, it is possible that the microorganisms having the ability to generate hydrogen and/or formic acid coexist. Examples of such microorganisms include Escherichia coli and Butyricimonas genus, with preferable strains being Escherichia coli K12, Butyricimonas virosa JCM15149, Butyricimonas synergistica JCM15184, Butyricimonas paravirosa JCM18677 and the like.

The preferable examples of the microorganisms having the ability to transform the compound represented by the formula (II) into the compound represented by the formula (I) include Flavonifractor genus bacteria (old scientific name; Eubacterium genus bacteria and Clostridium genus bacteria), with preferable strains being Flavonifractor plautii (old scientific name; Eubacterium plautii) strain ATCC29863, Flavonifractor plautii (old scientific name; Eubacterium plautii) strain MT42 (accession number FERM P-21765), and Flavonifractor plautii (old scientific name; Clostridium orbiscindens) strain ATCC49531.

The composition containing the compound represented by the formula (I) can be obtained easily by adding catechins and/or the composition containing catechins as a substrate in the cultured cell suspension or the culture solution in which the microorganisms having the ability to transform catechins mentioned above into the compound represented by the formula (II) and the microorganisms having the ability to transform the compound represented by the formula (II) into the compound coexist to incubate under anaerobic conditions. The incubation treatment methods include the method in which the cultured cells collected after culturing the microorganisms mentioned above are suspended in buffer, physiological saline, water and the like and the substrate is added in the suspension or the method in which during the culturing the microorganisms mentioned above or when a certain period of time has passed since the start of the culture the substrate is added. In addition the compound of the formula (I) can be obtained easily by adding the substrate in the culture solution in which the microorganisms mentioned above grow to incubate under the anaerobic conditions.

When the microorganisms mentioned above are cultured, the microorganisms are inoculated into the culture medium containing nutritional source in which the microorganisms can grow and cultured under the anaerobic conditions. The general culture method of the anaerobic microorganisms can be adopted for the microorganisms culture to obtain the cultured cells and the microorganisms culture in the presence of the substrate. When the incubation treatment is performed in the presence of the substrate after collecting the cultured cell, it is preferably performed under the anaerobic conditions. The culture medium used for culture is not particularly limited as long as it is the culture medium in which the microorganisms mentioned above can grow and for example, GAM broth (Nissui Pharmaceutical Co., Ltd. (now Shimazdzu Diagnostics Corporation)) and the like can be used.

The culture condition can be selected appropriately within the range in which the microorganisms mentioned above can grow. Generally, the culture conditions are pH 6.0 to 7.5 and the temperature 35 to 40°C, preferably pH 6.5 to 7.3 and the temperature 37 to 39°C. The culture time is generally 24 to 120 hours, preferably 48 to 72 hours. The various culture conditions mentioned above can be changed according to the type and the characteristics of the microorganisms used, the external conditions and the like appropriately and the optimum condition can be selected.

When the catechin metabolites are produced by the microorganism transformation, the extract containing several types of the catechin metabolites is obtained in preparation and the catechin metabolites having high purity can be obtained through repeated purification. Various known purification methods are selected and combined similarly to the organic chemical synthesis method to achieve the desired purity. By using for example, the solvent extraction using ethyl acetate, diethyl ether, butanol, and the like, the methods that utilize the adsorption and desorption of synthetic resin adsorbents, column chromatography using silica gel and the like, and high performance liquid chromatography alone or in combinations of two or more the separation and the purification are performed to control the content of the catechin metabolites in the extract.

The present invention is now described in more detail with reference to Manufacturing Examples of the compound of the formula (i) and Examples as follows and is not limited to these Examples.

### Examples

### <Manufacture of Compound of Formula (I)>

### (Manufacturing Example 1: manufacture of (R)-5-(3,5-dihydroxyphenyl)-γ-valerolactone under the coexistence of Eggerthella lenta strain JCM9979, Flavonifractor plautii strain ATCC49531 and Escherichia coli K12)

Eggerthella lenta strain JCM9979 was inoculated in 30 mL of GAM broth (Nissui Pharmaceutical Co., Ltd. (now Shimadzu Diagnostics Corporation)) and cultured at pH 7.2 and 37°C for 48 hours anaerobically to obtain preculture solution 1. Escherichia coli K12 and Eubacterium plautii strain ATCC49531 were cultured at pH 7.2 and 37°C for 24 hours anaerobically using 10 mL of GAM broth to obtain preculture solution 2. 30 mL of the preculture solution 1 of strain JCM9979 and 10 mL of the preculture solution 2 of Escherichia coli K12 and strain ATCC49531 were added in 100 mL of GAM broth containing 290 mg of (-)-epigallocatechin and cultured at pH 7.2 and 37°C for 72 hours anaerobically. 1 mL of the culture solution obtained was sampled and subjected to high-speed centrifugation (15000×g, 10 min, 4°C) to remove the cells. The supernatant was analyzed under the following LC/MS analytical conditions to confirm the production of 5-(3,5-dihydroxyphenyl)-γ-valerolactone and 5-(3,5-dihydroxyphenyl)-4-hydroxyvaleric acid. The LC/MS analytical conditions are described below.

### (LC/MS analytical conditions)

·column: Capcellpak C18 MG (2.0i.d.×100.0 mm, 5µm (manufactured by Shiseido Company, Limited))
·flow rate: 0.2 mL/min
·temperature of column: 40°C
·solvent
   solvent A: water: acetonitrile: acetic acid (100:2.5:0.1 volume ratio (v/v/v))
   solvent B: water: acetonitrile: methanol: acetic acid (35:2.5:65:0.1) volume ratio (v/v/v/v))
·gradient condition: 0 min A; 100 % B; 0 %, 3 min A; 100 % B; 0 % 25 min A; 0 % B; 100 % 25.1 min A; 100 % B; 0 % 33 min A; 100 % B; 0 %
·detector: PDA and mass spectrometer
·interface: ESI
·polarity: negative

The culture solution was subjected to high-speed centrifugation (10000×g, 20 min, 20°C) to remove the cells. 5M hydrochloric acid solution was added to the supernatant obtained to adjust to pH 2.0. By maintaining 5-(3,5-dihydroxyphenyl)-4-hydroxyvaleric acid in the supernatant at 80°C for about 2 hours, it was dehydrated and condensed to transform into 5-(3,5-dihydroxyphenyl)-γ-valerolactone. The reaction solution was extracted with 200 mL of ethyl acetate three times and the ethyl acetate phases were united and concentrated with an evaporator. The concentrated solution obtained was subjected to preparative HPLC. The preparative HPLC conditions were described below.

### (Preparative HPLC conditions)

·column: Capcellpak MG (2.0i.d.×150 mm, 5µm (manufactured by Shiseido Company, Limited))
·flow rate: 15 mL/min
·solvent
   solvent A: acetonitrile: methanol: water: acetic acid (5:5:90:0.3 volume ratio (v/v/v))
   Solvent B: acetonitrile: methanol: water: acetic acid (5:65:30:0.5 volume ratio (v/v/v))
·gradient condition: 0 min A; 80 % B; 20 %, 5 min A; 80% B; 20 % 20 min A; 10 % B; 90 % 25 min A; 10 % B; 90 % 26 min A; 80 % B; 20 % 35 min A; 80 % B; 20 %
·detector: UV270 nm

After performing preparative HPLC, by analyzing under the LC/MS conditions mentioned above the fractions containing the target metabolite were confirmed. Then the fractionated solution was concentrated to dryness with an evaporator. 5 mL of pure water was added to the dried product and the operation of concentrating to dryness was repeated three more times to completely remove acetic acid from the fraction. At the last, what is obtained by adding a small amount of pure water to the dried product to dissolve was freeze-dried to obtain 45 mg of 5-(3,5-dihydroxyphenyl)-γ-valerolactone.

### <Example 1 Effect of Intraperitoneal Administration of EGC-M5 on Composition of Dendritic Cells Formed in Bone Marrow>

### (a) Rearing Procedure and Administering Procedure

After preliminary rearing, 6-week-old male C57BL/6J mice were assigned to two groups of the administration group of EGC-M5 and Control group (10 mice per group). The mouse in the administration group of EGC-M5 was intraperitoneally administered with the test solution obtained by dissolving EGC-M5 in 10% DMSO PBS buffer at the dose of 10mg/kg b. w. of EGC-M5 every day. The mouse in Control group was intraperitoneally administered with 10% DMSO PBS buffer every day. After administering for 12 days, the mouse was euthanized by collecting blood under isoflurane anesthesia and the femur was collected.

### (b) Preparation of Bone Marrow Cells

The femur collected was placed on the 5 mL dish containing RPMI-1640 culture medium (FUJIFILM Wako Pure Chemical Corporation) and the bone marrow cells were collected with 2.5 mL syringe and 26G needle. The pieces of tissue were removed by filtering with the mesh. After the centrifugation of 5 min the number of the cells was counted using the hemocytometer.

### (c) Composition Analysis of each Dendritic Cell

The bone marrow cells were prepared to be 1.0×10⁷ cells/mL with RPMI-1640 (no Phenol Red) culture medium (FUJIFILM Wako Pure Chemical Corporation), mixed with an equivalent amount of 2 % PFA (obtained by diluting 4 % paraformaldehyde phosphate buffer (FUJIFILM Wako Pure Chemical Corporation) with sterile water for injection) and then maintained at 4°C for 20 min. After the centrifugation of 5 min, the supernatant was removed and the pellet was resuspended in 5% FBS-TPBS (0.2% Tween- PBS (FUJIFILM Wako Pure Chemical Corporation)). The suspension was dispensed to the 96 well with V bottom plate at 50 µL/well and maintained at the room temperature for 30 min. Each antibody shown in Table 1 was added at 50 µL/well and maintained at the room temperature for 1 hour to label cells fluorescently. Then 50 µL of the cell suspension and 250 µL of PBS were mixed. The proportion (%) of cDC1, cDC2, and pDC in the dendritic cells was measured with the flow cytometry. In the measurements the cells which were CD3 negative and CD11c positive based on the fluorescence intensity were sorted as "the dendritic cells". Among "the dendritic cells" the cells which were CD103 positive and I-A/I-E (MHC-II) strong positive were defined as "cDC1", the cells which were CD11b positive and I-A/I-E (MHC-II) strong positive were defined as "cDC2" and the cells which were B220 positive and I-A/I-E (MHC-II) weakly positive were defined as "pDC".

The antibodies used for the measurement of each dendritic cell (cDC1, cDC2, and pDC) by the flow cytometry were shown in Table 1.

| cell type | antibody manufactured by Biolegend, inc. | volume (in 50 *µ* l 5%FBS-TPBS) |
|---|---|---|
| cDC1 | FITC anti-mouse CD3 | 5 *µ* l |
| | PE anti-mouse CD11c Antiboby | 4 *µ* l |
| | APC anti-mouse I-A/I-E | 0.75 *µ* l |
| | PreCP/Cyanine5.5 anti-mouse CD103 Antibody | 4 *µ* l |
| cDC2 | FITC anti-mouse CD3 | 5 *µ* l |
| | PE anti-mouse CD11c Antiboby | 4 *µ* l |
| | APC anti-mouse I-A/I-E | 0.75 *µ* l |
| | PreCP anti-mouse/human OD11b | 4 *µ* l |
| pDC | FITC anti-mouse CD3 | 5 *µ* l |
| | PE anti-mouse CD11c Antiboby | 4 *µ* l |
| | APC anti-mouse I-A/I-E | 0.75 *µ* l |
| | Brilliant Violet 510 anti-mouse/human CD45R/B220 Antibody | 1 *µ* l |

### (d) Statistical Processing Method

From the results obtained, the mean (Mean) and the standard error (S.E.) were calculated. The significant difference between Control group and the EGC-M5 administration group was assessed by Student's t-test. The level of significance was set to *P < 0.05.

### (e) Test Result

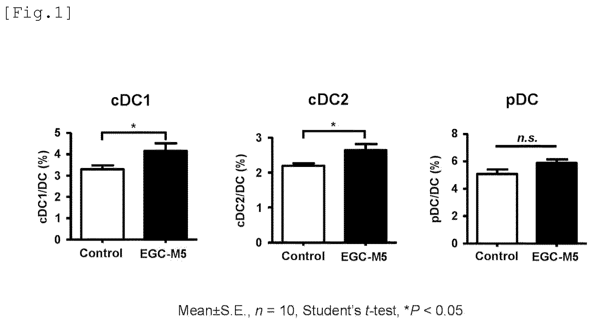

As shown in Fig.1 regarding the dendritic cells formed in mouse bone marrow it was shown that the proportion of conventional dendritic cells (cDC1s and cDC2s) increased significantly compared with Control group and that the proportion of plasmacytoid dendritic cells (pDCs) did not change in the EGC-M5 administration group.

From this result it was shown that the administration of EGC-M5 had the action of promoting the differentiation into conventional dendritic cells (cDC1s and cDC2s) during the process of the differentiation of hematopoietic stem cells into dendritic cells within the bone marrow in the body.

### <Example 2 Effect of EGC-M5 on Differentiation and Activation of Bone Marrow-derived Precursor Dendritic Cells (BMDCs)>

### (a) Used Animal and Rearing Procedure

After preliminary rearing 6-week-old male C57BL/6J mice were euthanized by collecting blood under isoflurane anesthesia and the femur was collected.

### (b) Preparation of Bone Marrow Cells

Bone marrow cells were isolated from the femur in the manner similar to the manner described in (b) of Example 1.

### (c) Differentiation of Bone Marrow-derived Dendritic Cells (BMDCs) in the Presence of EGC-M5

Bone marrow cells including bone marrow-derived precursor dendritic cells (BMDCs) were prepared to be 2.0×10⁵ cells/mL with 0, 1, and 10 µM EGC-M5 R10 culture medium (10 % heat inactivated FBS, 20 ng/mL GM-CSF, 50 µM 2-mercaptoethanol RPMI) and inoculated in 10 mL dish for suspension cells. After culturing at 37°C in the presence of 5 % CO₂ for 72 hours, 10 mL of 0, 1, and 10µM EGC-M5 R10 culture medium were added. After culturing for 48 hours 10 mL of the culture supernatant was collected and subjected to centrifugation. The pellet was suspended in 10 mL of 0, 1, and 10 µM EGC-M5 R10 culture medium and returned to the original dish. After culturing for 48 hours, precursor dendritic cells (BMDCs) were differentiated into immature dendritic cells by performing the treatment similar to the treatment performed just before. After additionally culturing for 48 hours the cells were collected and resuspended in 0, 1, and 10 µM EGC-M5 maturation induction medium (10 % heat inactivated FBS, 10 ng/mL GM-CSF, 50 µM 2-mercaptoethanol, 1 µg/mL LPS). The cells were inoculated in 10 mL dish for adherent cells and immature dendritic cells were changed into mature dendritic cells (activated cDC). After culturing for 24 hours the cells and the culture supernatant were collected.

### (d) Composition Analysis of Each Dendritic Cell

The cells obtained by the operation of (c) were collected and the proportions of "cDC1", "cDC2" "pDC" and "activated pDC" in the dendritic cells were measured with the flow cytometry similarly to (c) of Example 1. The cells which were I-A/I-E (MHC-II) positive among "the dendritic cells" were defined as "the activated DC". The antibodies used for the measurement of "the activated DC" were as shown in Table 2.

The antibodies used for the measurement of the activated DCs (MHC-II⁺DCs) with the flow cytometry were shown in Table 2.

| cell type | antibody manufactured by Biolegend, inc. | volume (in 50µl 5%FBS-TPBS) |
|---|---|---|
| MHC-II+DC | FITC anti-mouse CD3 | 5 *µ* l |
| | PE anti-mouse CD11c Antiboby | 4 *µ* l |
| | APC anti-mouse I-A/I-E | 0.75 *µ* l |

### (e)Analysis of IL-12 Production Amount and Expression Level of cDC and pDC Genes

The concentration of IL-12 in the culture supernatant obtained by the operation of (c) was measured by ELISA method. Mouse IL-12 p70 DuoSet ELISA (DY419-05, R&D Systems Inc.) and DuoSet Ancillary Reagent Kit 2,5 Plate (DY008, R&D Systems Inc.) were used for the measurement. RNA of the cells obtained by the operation of (c) was extracted and cDNA was synthesized using Prime Script^{™} RT Reagent Kit (TAKARA BIO INC.). The synthesized cDNA, PCR primers for detecting each gene, and SsoAdvanced Universal SYBR Green Supermix (BIO-RAD Laboratories, Inc.) were mixed and each gene expression level was measured by real-time PCR method. Actb was used as internal control gene. The primer sequence of each gene was described below.
(cDC1 marker)
   Xcr1: Forward 5'- ACATGATACCCATGGGGAAGT -3'
      Reverse 5'- GTGCACGAAGTGTTGCTTTG -3'
   Cd103: Forward 5'- GCCGTGATCCAGACTGAGTTTGAT -3'
      Reverse 5'- ATGGCTGAGGCGGTCTTAGTGACT -3'
(cDC2 marker)
   Cd11b: Forward 5'- CCACTCATTGTGGGCAGCTC -3'
   Reverse 5'- GGGCAGCTTCATTCATCATGTC -3'
(pDC marker)
   Bst2: Forward 5'- ACATGGCGCCCTCTTTCTATCACT -3'
   Reverse 5'- TGACGGCGAAGTAGATTGTCAGGA -3'
(cytokine IL-12 gene)
   Il-12a: Forward 5'- TCTGGTACATCTTCAAGTCCTCATAGA -3'
   Reverse 5'- TACTAGAGAGACTTCTTCCACAACAAGAG -3'
(internal control gene)
   Actb: Forward 5'- CATCCGTAAAGACCTCTATGCCAA-3'
   Reverse 5'- ATGGAGCCACCGATCCACA -3'

### (f) Statistical Processing Method

From the results obtained, the mean (Mean) and the standard error (S.E.) were calculated. The significant differences between Control group and the groups treated with EGC-M5 were assessed by Dunnett's test. The level of significance was set to *P < 0.05 and **P < 0.01.

### (g) Test Result

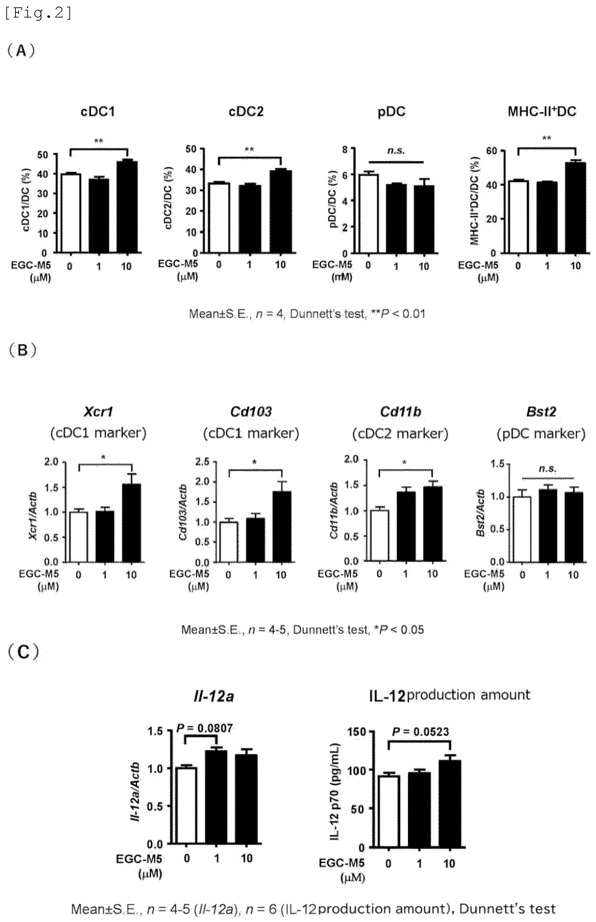

The result of the composition analysis of each dendritic cell in (d) showed that the proportion of cDC1s and cDC2s in the dendritic cells increased significantly compared with Control group, whereas the proportion of pDCs did not change and that on the other hand the proportion of the activated DCs (MHC-II⁺DCs) increased significantly in the group in which precursor dendritic cells (BMDCs) were treated with 10 µM EGC-M5 (Fig.2 (A)).

Next, the result of the analysis of the gene expression level of the cDC1, cDC2, and pDC markers in (e) showed that the gene expression level of cDC1 marker (XCR1, CD103), cDC2 marker (CD11b) increased significantly compared with Control group and that the significant fluctuation of the gene expression level of pDC marker (BST2) was not observed in the group treated with 10 µM EGC-M5 (Fig.2 (B)).

Additionally, the result of cytokine IL-12 gene expression level and the production amount of IL-12 in (e) showed the tendency that IL-12 gene expression level increased in the group treated with 1 µM EGC-M5 and that the production amount of IL-12 increased in the group treated with 10 µM EGC-M5 (Fig.2 (C). Because IL-12 is cytokine which the activated cDC1s mainly secrete, it was considered that the increase of IL-12 gene expression level and IL-12 production amount is caused by the increase of the activated cDC1s.

From the results above, it was shown that EGC-M5 promoted the differentiation into the conventional dendritic cells (cDC1s and cDC2s) and at the same time activated the immature cDCs to change into the mature cDCs by directly acting on the precursor dendritic cells (BMDCs) .

The results of Examples 1 and 2 showed the possibility that the intraperitoneal administration and the addition to bone marrow-derived precursor dendritic cells of EGC-M5 promoted the differentiation of precursor dendritic cells into cDC1s and cDC2s and activated cDCs. Then the effect of EGC-M5 on Th1 cells and cytotoxic T cells (CTLs) which were promoted to differentiate and activated by cDC1s and cDC2s was evaluated in the subsequent tests.

### <Effect of EGC-M5 on Activation of Th1 Cells in Mouse Splenic Cells>

### (a) Rearing Procedure

After preliminary rearing, 6-week-old male C57BL/6J mice were euthanized by collecting blood under isoflurane anesthesia and the spleen was collected.

### (b) Analysis of T Cell Activation-related Genes Expression Level in Splenic Cells in the Presence of EGC-M5

The spleen collected was placed in 5 mL dish containing RPMI-1640 culture medium and the tissue was ground with the slide glass to disperse the cells. The tissue pieces were removed by filtering with the mesh and the filtrate was subjected to the centrifugation for 5 min. After the supernatant was removed, the hemolysis treatment was performed with BD Pharm Lyse^{™} Lysing Buffer (Becton, Dickinson and Company) . Note that Lysing Buffer was diluted 10 times with the sterile water for injection (Otsuka Pharmaceutical Factory, Inc.) for use. After the cells were washed with RPMI-1640 culture medium 2 times, the number of cells was measured using the hemocytometer. The cells were prepared to be 1.0×10⁷ cells/mL with 10 % FBS-RPM1640 culture medium and inoculated in the 24 well plate. Then EGC-M5 was added to make the final concentration 0, 1, and 10 µM. After culturing at 37°C in the presence of 5 % CO₂ for 72 hours, RNA was extracted using RNeasy Mini kit (QIAGEN N.V.) and cDNA was synthesized using Prime Script^{™} RT Reagent Kit (TAKARA BIO INC.). The synthesized cDNA, the respective primers, and SsoAdvanced Universal SYBR Green Supermix (BIO-RAD Laboratories, Inc.) were mixed and Th1 cell activation-related gene expression levels were measured by real-time PCR method. Actb was used as internal control gene.

Il-12a is a cytokine gene that activated cDC1s mainly produce, and IL-12 acts on naive CD4⁺ T cells to differentiate into Th1 cells. T-bet is a transcription factor which is expressed by the activation of the signal transduction pathways when cytokines such as IL-12 bind to the receptor on naive CD4⁺ T cells. T-bet enhances the expression of IFN-γ gene and promotes the differentiation of naive CD4⁺ T cells into Th1 cells. It is known that TNF-α and IFN-γ are cytokines which Th1 cells secrete and induce CTL activation.

The primer sequence of each gene was described below.
(cytokine IL-12 gene)
   Il-12a: Forward 5'- TCTGGTACATCTTCAAGTCCTCATAGA -3'
   Reverse 5'- TACTAGAGAGACTTCTTCCACAACAAGAG -3'
(Th1 cell differentiation-related gene)
   T-bet: Forward 5'- AACCAGTATCCTGTTCCCAGC -3'
   Reverse 5'- TGTCGCCACTGGAAGGATAG -3'
(cytokine TNF-α gene)
   Tnf-α: Forward 5'- GAGGCACTCCCCCAAAAGAT -3'
   Reverse 5'- CGATCACCCCGAAGTTCAGT -3'
(cytokine IFN-γ gene)
   Ifn-y: Forward 5'-GCTTCCTGAGGCTGGATTC-3'
   Reverse 5'-GGATGCATTCATGAGTATTGG-3'
(internal control gene)
   Actb: Forward 5'- CATCCGTAAAGACCTCTATGCCAA-3'
   Reverse 5'- ATGGAGCCACCGATCCACA -3'

### (c) Evaluation of IFN-γ production amount

After isolating the splenic cells from the spleen similarly to (b), the cells were prepared to be 1.0×10⁷ cells/mL with 4 µg/mL PHA 10 % FBS-RPMI1640 culture medium (FUJIFILM Wako Pure Chemical Corporation) and inoculated in the 96 well plate. Then EGC-M5 was added to make the final concentration 0, 1, and 10 µM. After culturing at 37°C in the presence of 5 % CO₂ for 72 hours, the culture supernatant was collected and IFN-γ production amount was measured by ELISA method. The antibody set of Mouse IFN-gamma DuoSet ELISA (DY485-05, R&D Systems Inc.) and the optional kit of DuoSet Ancillary Reagent Kit 2,5 Plate (DY008, R&D Systems Inc.) were used for the measurement.

### (d) Statistical Processing Method

From the results obtained, the mean (Mean) and the standard error (S.E.) were calculated. The significant differences between Control group and the groups treated with EGC-M5 were assessed by Dunnett's test. The level of significance was set to * P < 0.05 and ** P < 0.01.

### (e) Test Result

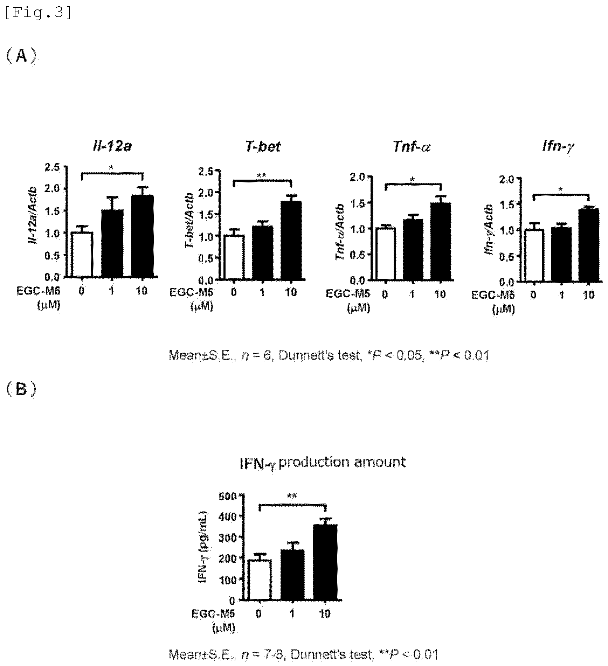

As shown in Fig.3 IL-12a, T-bet, TNF-α, and IFN-γ gene expression levels (Fig.3 (A)) and IFN-γ production amount (Fig.3 (B)) in the splenic cells significantly increased in the group treated with 10 µM EGC-M5 compared with Control group. The increase in IL-12a gene and T-bet gene expression levels showed that EGC-M5 promoted the differentiation of naive CD4+ T cells into Th1 cells. The increase in TNF-α, and IFN-γ gene expression level showed that EGC-M5 enhanced the ability to produce cytokines which Th1 cells secrete. The increase in IFN-γ production amount showed that EGC-M5 had the action of enhancing the cytotoxic activity of the cytotoxic T cells (CTLs).

These results showed that EGC-M5 promoted the differentiation of naive CD4⁺ T cells into Th1 cells in splenic cells and enhanced the ability to produce cytokines which Th1 cells secrete, thereby enhancing the cytotoxic activity of cytotoxic T cells (CTLs) on which the produced IFN-γ acts, thereby strengthening cellular immunity.

### <Example 4: Effect of Intraperitoneally Administering EGC-M5 to Colon Cancer Model Mouse on Activation of Th1 Cells in Splenic Cells>

### (a) Rearing Procedure and Administering Procedure

After 6-week-old male C57BL/6J mice were preliminary reared, mouse colon cancer cell strain MC38 cells were subcutaneously transplanted at 1.0 × 10⁶ cells/mouse. 4 days after transplantation the mice were assigned to two groups of the administration group of EGC-M5 and Control group (10 mice per group). The mouse in the administration group of EGC-M5 was intraperitoneally administered with 200 µL of 10 % DMSO PBS buffer containing EGC-M5 prepared to make the dose 10 mg/kg b. w. of EGC-M5 every day. The mouse in Control group was intraperitoneally administered with 10 % DMSO PBS buffer every day. 16 days after transplantation the mouse was euthanized by collecting blood under isoflurane anesthesia and the spleen and the tumor tissue were collected.

### (b) Analysis of T Cell Differentiation-related Cytokine Gene Expression Levels

The spleen collected was immersed in 5 mL of RPMI1640 culture medium in 5 mL dish. Next, the spleen was ground with the frosted glass slides and filtered with the 70 µm cell strainer (FALCON, Corning Inc.). After centrifugation at 2,500 rpm for 5 min, the supernatant was removed. After suspending with 1 mL of Lysing Buffer (manufactured by Becton, Dickinson and Company) , the suspension was maintained on the ice for 1 min. The reaction was stopped by adding 2 mL of PBS. After centrifugation at 2,500 rpm for 5 min, the supernatant was removed and the pellet was suspended in 10 mL of RPMI1640 culture medium. The suspension was centrifuged at 2,500 rpm for 5 min again and the supernatant was removed. Then the pellet was suspended in RPMI1640 culture medium and the number of cells was measured. RNA in the cells was extracted using RNeasy Mini kit (QIAGEN N.V.) and cDNA was synthesized using Prime Script^{™} RT Reagent Kit (TAKARA BIO INC.). The synthesized cDNA, the respective primers, and SsoAdvanced Universal SYBR Green Supermix (BIO-RAD Laboratories, Inc.) were mixed and Th1 cell, Th2 cell activation-related gene expression was evaluated by real-time PCR method. Actb was used as internal control gene. The primer sequence of each gene was described below.
(cytokines genes)
   Il-12a: Forward 5'- TCTGGTACATCTTCAAGTCCTCATAGA -3'
      Reverse 5'- TACTAGAGAGACTTCTTCCACAACAAGAG -3'
   Il-12b: Forward 5'- AACTTGAGGGAGAAGTAGGAATGG -3'
      Reverse 5'- GGAAGCACGGCAGCAGAATA -3'
   Il-2: Forward 5'- CTTCAAGCTCCACTTCAAGCT -3'
      Reverse 5'- CCATCTCCTCAGAAAGTCCACC -3'
   Ifn-y: Forward 5'-GCTTCCTGAGGCTGGATTC-3'
      Reverse 5'-GGATGCATTCATGAGTATTGG-3'
   Il-4: Forward 5'- TCCTCACAGCAACGAAGAAC -3'
      Reverse 5'- CAAGCATGGAGTTTTCCCATG -3'
   Il-5: Forward 5'- TCAGCTGTGTCTGGGCCACT -3'
      Reverse 5'- TTATGAGTAGGGACAGGAAGCCTCA -3'
   Il-6: Forward 5'- GGCCTTCCCTACTTCACAAG -3'
      Reverse 5'- ATTTCCACGATTTCCCAGAG -3'
   Il-10: Forward 5'- GACCAGATGGACAACATACTGATAA -3'
      Reverse 5'- GACCAGCTGGACAACATACTGCTAA -3
(internal control gene)
   Actb: Forward 5'- CATCCGTAAAGACCTCTATGCCAA-3'
   Reverse 5'- ATGGAGCCACCGATCCACA -3'

### (c) Statistical Processing Method

From the results obtained, the mean (Mean) and the standard error (S.E.) were calculated. The significant difference between Control group and the administration group of EGC-M5 was assessed by Student's t-test. The level of significance was set to * P < 0.05 and ** P < 0.01.

### (d) Test Result

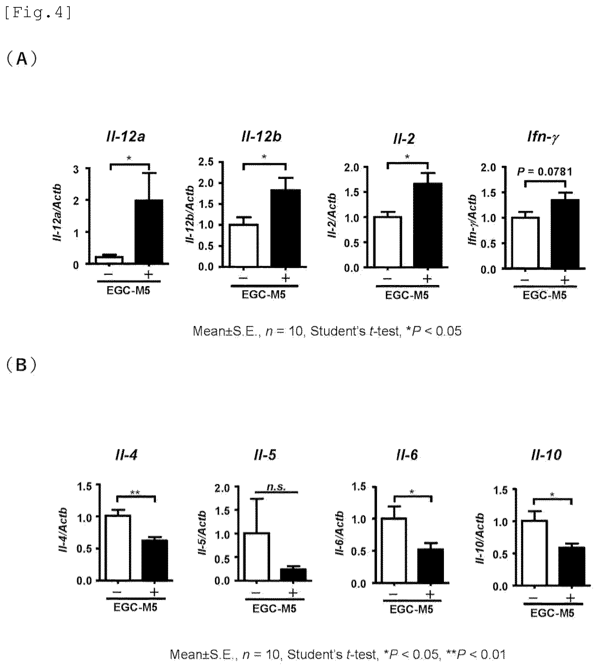

As shown in Fig.4 the expression level of the genes of IL-12a and IL-12b inducing the differentiation of the naive CD4+ T cells into Th1 cells in the spleen of colon cancer model mouse increased in the administration group of EGC-M5. The expression level of the genes of cytokines IL-2 and IFN-γ which Th1 cells secrete also increased (Fig.4 (A)). On the other hand, the expression level of the genes of cytokines Il-4, Il-6, and Il-10 produced by Th2 cells having the action opposite to that of Th1 cells decreased in the administration group of EGC-M5 (Fig.4(B)).

From this result it was shown that administering EGC-M5 to colon cancer model mouse promoted the differentiation of naive CD4⁺ T cells into Th1 cells and inhibited the differentiation into Th2 cells having the action opposite to that of Th1 cells (Fig.7 e)

### <Example 5: Effect of Intraperitoneally Administering EGC-M5 to Colon Cancer Model Mouse on Activation of Cytotoxic T Cells (CTLs) and Tumor Infiltration>

### (a) Evaluation of Effect of EGC-M5 on Activation of CTLs

The spleen collected in Example 4 was immersed in 5 mL of RPMI1640 culture medium in 5 mL dish. Next, the spleen was ground with the frosted glass slides and filtered with the 70 µm cell strainer (FALCON, Corning Inc.). After centrifugation at 2,500 rpm for 5 min, the supernatant was removed. After suspending with 1 mL of Lysing Buffer (manufactured by Becton, Dickinson and Company) , the suspension was maintained on the ice for 1 min. The reaction was stopped by adding 2 mL of PBS. After centrifugation at 2,500 rpm for 5 min, the supernatant was removed and the pellet was suspended in 10 mL of RPM1640 culture medium. The suspension was centrifuged at 2,500 rpm for 5 min again and the supernatant was removed. Then the pellet was suspended in RPMI1640 culture medium and the number of cells was measured. RNA in the cells was extracted using RNeasy Mini kit (QIAGEN N.V.) and cDNA was synthesized using Prime Script^{™} RT Reagent Kit (TAKARA BIO INC.). The synthesized cDNA, the respective primers, and SsoAdvanced Universal SYBR Green Supermix (BIO-RAD Laboratories, Inc.) were mixed and CTL activation-related gene expression was evaluated by real-time PCR method. Actb was used as internal control gene. The primer sequence of each gene was described below.
Perforin: Forward 5'- GAGAAGACCTATCAGGACCA -3'
   Reverse 5'- AGCCTGTGGTAAGCATG -3'
Grzb: Forward 5'- CCTCCTGCTACTGCTGAC -3'
   Reverse 5'- GTCAGCACAAAGTCCTCTC -3'
Actb: Forward 5'- CATCCGTAAAGACCTCTATGCCAA-3'
   Reverse 5'- ATGGAGCCACCGATCCACA -3'

### (b) Evaluation of Infiltration of CD8⁺ T Cells in Tumor Tissue

The tumor tissue collected in Example 4 was immersed in 4 % paraformaldehyde for 1 week. Then the production of the paraffin block and the tissue slices were entrusted to KYODO BYORI Inc.. After performing deparaffinization using Lemosol (FUJIFILM Wako Pure Chemical Corporation), heat treatment was performed with immunosaver (NISSHIN EM Co., LTD.) to activate antigens. After blocking by treating with 5 % FBS-Sodium azide (0.01 % sodium azide-PBS (FUJIFILM Wako Pure Chemical Corporation)) at the room temperature for 1 hour, the treatment with anti-CD8 alpha antibody (abcam. plc, ×200) was performed at 4°C overnight. Then staining was performed by treating with Alexa Flour488 F(ab')2 fragment of goat anti-IgG (Thermo Fisher Scientific Inc., ×500), Hoechst 33342, trihydrochloride trihydrate (H3570, Thermo Fisher Scientific Inc., ×10000) at 4°C for 1 hour. After enclosing the tissue with VECTASHIELD (Vector Laboratories, Inc.) and the cover glass, the number of the green fluorescent cells (CD8⁺ T cells) per one area was counted visually by observing with all-in-one fluorescence microscope BZ-X700 (KEYENCE CORPORATION).

### (c) Statistical Processing Method

From the results obtained, the mean (Mean) and the standard error (S.E.) were calculated. The significant difference between Control group and the administration group of EGC-M5 was assessed by Student's t-test. The level of significance was set to ** P < 0.01.

### (e) Test Result

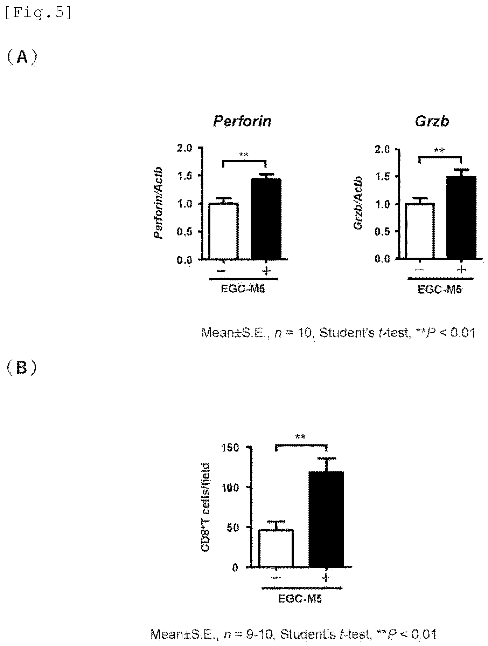

As shown in Fig.5(A), the expression of Perforin and Grzb, which were the genes of the cytotoxic substance produced by the cytotoxic T cells (CTLs) in the splenic cells, increased by intraperitoneally administering EGC-M5 to the colon cancer model mouse. In addition, by administering EGC-M5 the remarkable accumulation of the CD8⁺ T cells (CTLs) in the tumor was observed and the number of the CD8⁺ T cells per one area significantly increased by administering EGC-M5 (Fig.5 (B)). These results showed the possibility that EGC-M5 exerted the action of activating CTLs and promoting the infiltration of cells into the tumor tissue and increased the attack capability of the CTLs against cancer cells (enhanced the cellular immunity).

### Industrial Applicability

The present dendritic cell activator, the present medicine, the present supplement, or the present food and beverage, which promote the differentiation into the conventional dendritic cells which induce or promote the acquired immune response and activate the conventional dendritic cells, are useful in the field of preventing or treating the cancer or infectious disease.

## Claims

1. A dendritic cell activator **characterized by** containing, as an active ingredient, 5-(3,5-dihydroxyphenyl)-γ-valerolactone represented by formula (I); 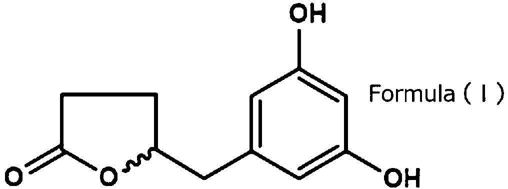 wherein in formula (I), the configuration of the wavy line may be either R-form or S-form.

2. The dendritic cell activator according to Claim 1, used for promoting differentiation into conventional dendritic cells (cDCs).

3. The dendritic cell activator according to Claim 2, wherein promotion of the differentiation into the conventional dendritic cells (cDCs) is accompanied by activation of the conventional dendritic cells (cDCs).

4. The dendritic cell activator according to Claim 2, wherein the dendritic cell activator promotes differentiation of CD4⁺ T cells into Th1 cells and inhibits differentiation of CD4⁺ T cells into Th2 cells

5. The dendritic cell activator according to Claim 2, wherein the dendritic cell activator promotes differentiation of CD8⁺ T cells into cytotoxic T cells (CTLs) and enhances cytotoxic activity.

6. The dendritic cell activator according to any one of Claims 1 to 5, wherein the dendritic cell activator is an enhancer of acquired immune system.

7. The dendritic cell activator according to Claim 6, wherein the acquired immune system is cellular immunity.

8. The dendritic cell activator according to Claim 6, wherein the dendritic cell activator is an anticancer drug.
